# EUROPEAN PATENT APPLICATION

(11) **EP 3 660 163 A1**
(43) Date of publication of application: **03.06.2020**
(21) Application number: 17919474.1
(22) Date of filing: 08.08.2017
(51) Int. Cl.: C12Q 1/68

(54) **NUCLEIC ACID HOMOGENIZATION METHOD, AND KIT AND USE THEREOF**

(30) Priority: 26.07.2017 CN 201710618321
(71) Applicant: Shanghai Zj Bio-tech Co., Ltd., Pudong, Shanghai 201114 (CN)
(72) Inventor: ZHANG, Jie, Shanghai 201114 (CN); LI, Qiang, Shanghai 201114 (CN); WANG, Kai, Shanghai 201114 (CN); WANG, Ning, Shanghai 201114 (CN); NI, Jingyi, Shanghai 201114 (CN); LU, Zhongying, Shanghai 201114 (CN); SHAO, Junbin, Shanghai 201114 (CN)
(74) Representative: Baudler, Ron
(86) International application number: PCT/CN2017/096420
(87) International publication number: WO 2019/019219

(57) **Abstract**

The present invention provides a nucleic acid homogenization method, and a kit and use thereof, the method comprising at least the following steps: respectively adding nucleic acid adsorption materials having the same nucleic acid saturation adsorption amount into several nucleic acid solutions, and ensuring that the nucleic acid adsorption materials added into each nucleic acid solution can all achieve a nucleic acid adsorption saturation state; separating the nucleic acid adsorption materials saturated with nucleic acids; respectively eluting the nucleic acids from the isolated nucleic acid adsorption materials. The nucleic acid homogenization method disclosed in the present invention allows for the dilution of a nucleic acid, a PCR product or a high throughput sequencing library concentration in an equal dilution ratio.

## Description

### Technical field

The present disclosure belongs to the technical field of high throughput sequencing, and specifically relates to a nucleic acid homogenization method, and a kit and use thereof. The nucleic acid homogenization method allows for rapid and stable equal proportional dilution of nucleic acid, a PCR product or a high throughput sequencing library concentration, therefore is suitable for the large-scale application.

### Background

Nucleic acid screening or diagnosis has been widely used in clinical, disease prevention and control, food safety testing, import and export quarantine detection and other scenarios. For some application scenarios, such as high throughput sequencing, in order to continuously analyze in parallel, increase the amount of experimental detection, and reduce the cost of sequencing, different samples are usually labeled and mixed together for sequencing. This requires the concentration of different samples is basically the same before mixing, and eventually different samples may produce the same amount of data.

Nucleic acid homogenization refers to the process of making several portions of nucleic acid into samples containing equal content of nucleic acid. Conventional equal proportional dilution of concentration of nucleic acid is usually realized by continuous dilution after quantification. Quantification is usually realized by absorbance, fluorescent dyes, or fluorescent probes. Absorbance quantification is usually significantly affected by other substances (such as proteins, polysaccharides, non-target fragment nucleic acids and other impurities), resulting in a large deviation in the quantification of the target products. The fluorescent dye method or fluorescent probe method is accurate, but it is complicated and time-consuming for series dilution of the samples is. Particularly, the introduction of the special fluorescence quantitative instruments (such as Qubit or various fluorescent quantitative PCR instruments) and corresponding reagents would greatly increase the cost of equal proportional dilution of nucleic acid samples. In addition, it is almost impossible to realize the automatic equal proportional dilution operation of a large number of samples, which has great limitations on the application scenarios with high timeliness requirements such as clinical practice and disease control.

Magnetic bead is a kind of nano-microspheres, widely applied in the extraction of nucleic acids. The binding of nucleic acid to magnetic beads mainly depends on electrostatic interaction, hydrophobic interaction, and hydrogen-bonding interaction. DNA or RNA releases from cells or tissues under the effect of lysate. At this time, the surface-modified super paramagnetic silica nano magnetic beads are "specifically bound" to the nucleic acid to form a "nucleic acid-magnetic bead composite". Then, under the effect of an external magnetic field, the composite is separated. Finally, after washing off the nonspecific adsorbed impurities by the eluent, desalting, and purifying, the nucleic acid substance is obtained.

However, how to realize the homogenization of nucleic acids by using magnetic beads or other nucleic acid adsorption materials has not been reported yet.

### Summary

The present disclosure provides a nucleic acid homogenization method, and a kit and use thereof, so as to solve the shortcomings of nucleic acid homogenization methods in the conventional technology, such as tediousness, large deviations, and difficult to automate.

The present disclosure provides a nucleic acid homogenization method, including at least the following steps:
(1) respectively adding nucleic acid adsorption materials having the same nucleic acid saturation adsorption amount into a plurality of nucleic acid solutions, the nucleic acid adsorption materials added into each nucleic acid solution achieves a nucleic acid adsorption saturation state;
(2) separating the nucleic acid adsorption materials of the saturation absorbed nucleic acids;
(3) eluting the nucleic acids from the separated nucleic acid adsorption materials.

Preferably, the nucleic acid adsorption materials are coated with any one or more of a carboxyl group, an amino group, a hydroxyl group, and a silicon group.

Preferably, the nucleic acid adsorption material added into each of the nucleic acid solutions is the same, and has the same amount.

Preferably, the nucleic acid adsorption materials are nano-microspheres or glass particles.

Preferably, the nucleic acid adsorption materials are monodisperse nano-microspheres or monodisperse glass particles.

Preferably, the nano-microspheres is capable of being magnetically adsorbed.

Preferably, the nano-microspheres are formed by coating Fe₃O₄ with oleic acid.

Preferably, the average particle diameter of the nano-microspheres is 0.5 to 2 µm.

Preferably, the method further includes a step (4): adding a solvent to the nucleic acid.

Further, the method includes a step (4): adding a solvent to the nucleic acid.

The solvent refers to a commonly used solvent for purifying and dispersing nucleic acids.

Another aspect of the present disclosure provides a kit, which includes a nucleic acid adsorption material. The particle material is coated with any one or more of a carboxyl group, an amino group, a hydroxyl group, or a silicon group.

Preferably, the nucleic acid adsorption materials are nano-microspheres or glass particles.

More preferably, the nucleic acid adsorption materials are monodisperse nano-microspheres or monodisperse glass particles.

More preferably, the nano-microspheres is capable of being magnetically adsorbed.

More preferably, the nano-microspheres are formed by coating Fe₃O₄ with oleic acid.

Preferably, the diameter of the particle material is 0.5 to 2 µm.

Preferably, the particle material is monodisperse.

Preferably, the kit further includes at least one of ethanol (volume fraction of 70-85%), ddH₂O or Tris-HCL buffer.

Preferably, the pH of the Tris-HCL buffer is 7.0-8.5.

Another aspect of the present disclosure provides the use of the above kit for a nucleic acid homogenization method.

As described above, the nucleic acid homogenization method of the present disclosure has the following beneficial effects:
By adopting the method of the present disclosure, the homogenization of nucleic acids can be achieved quickly. In particular, the equal proportional dilution of multiple nucleic acids can be realized fast with a small deviation, which is especially suitable for high throughput sequencing of nucleic acids.

### Detailed Description of the Preferred Embodiments

The implementation mode of the present disclosure will be described below through specific embodiments. Those skilled in the art can easily understand other advantages and effects of the present disclosure according to contents disclosed by the specification. The present disclosure can also be implemented or applied through other different specific implementation modes. Various modifications or changes can also be made to all details in the specification based on different points of view and applications without departing from the spirit of the present disclosure. It should be noted that processing equipment or devices not specifically noted in the following embodiments are all conventional equipment or devices in the field. In addition, it should be understood that one or more method steps mentioned in the present disclosure are not exclusive of other method steps that may exist before or after the combined steps or that other method steps may be inserted between these explicitly mentioned steps, unless otherwise stated; it should also be understood that the combined connection relationship between one or more equipment/devices mentioned in the present disclosure does not exclude that there may be other equipment/devices before or after the combined equipment/devices or that other equipment/devices may be inserted between these explicitly mentioned equipment/devices, unless otherwise stated. Moreover, unless otherwise stated, the numbering of each method step is only a convenient tool for identifying each method step, and is not intended to limit the order of each method step or to limit the scope of the present disclosure. The change or adjustment of the relative relationship shall also be regarded as the scope in which the present disclosure may be implemented without substantially changing the technical content.

In the present specification and claims, the singular forms "a", "an" and "the" include the plural forms, unless specifically stated otherwise.

When a range of numerical values is given by an embodiment, it is to be understood that the two endpoints of each range of numerical values and any value between the two endpoints may be selected unless otherwise stated. Unless otherwise defined, all technical and scientific terms used in the present disclosure have the same meaning as commonly understood by one skilled in the art. In addition to the specific method, equipment and material used in the embodiments, any method, equipment and material in the existing technology similar or equivalent to the method, equipment and material mentioned in the embodiments of the present disclosure may be used to realize the invention according to the grasp of the existing technology and the record of the invention by those skilled in the art.

Unless otherwise stated, the experimental methods, detection methods, and preparation methods disclosed in the present invention all employ conventional techniques of molecular biology, biochemistry, chromatin structure and analysis, analytical chemistry, cell culture, recombinant DNA technology in the technical field and related fields. These techniques are well described in the prior literature. For details, please see Sambrook et al. MOLECULAR CLONING: A LABORATORY MANUAL, Second edition, Cold Spring Harbor Laboratory Press, 1989 and Third edition, 2001; Ausubel et al., CURRENT PROTOCOLS IN MOLECULAR BIOLOGY, John Wiley & Sons, New York, 1987 and periodic updates; the series METHODS IN ENZYMOLOGY, Academic Press, San Diego; Wolfe, CHROMATIN STRUCTURE AND FUNCTION, Third Edition, Academic Press, San Diego, 1998; METHOD IN ENZYMOLOGY, Vol. 304, Chromatin (PMWassarman and APWolffe, eds.), Academic Press, San Diego, 1999; and METHODS IN MOLECULAR BIOLOGY, Vol. 119, Chromatin Protocols (PBBecker, ed.) Humana Press, Totowa, 1999, etc.

The reagents in the following embodiments are all commercially available.

### Embodiment 1 Preparation of Nano-microspheres

1.1 Under an atmosphere of nitrogen, adding excess aqueous ammonia to the Fe²⁺ / Fe³⁺ salt solution, and reacting for 0.5 hours at 80°C to obtain Fe₃O₄. Adding 20% oleic acid, incubating for 1.5 hours at room temperature, and then washing with ddH₂O until pH reaches 7.0. After the supernatant is removed by magnetic separation, washing with 100% ethanol for 3 times, and vacuum-drying at 40°C to obtain the nano-microsphere powder. Adding octane solution at 1: 1 w/v to obtain oleic acid-embedded Fe₃O₄ nano-microsphere mixture. Then, adding ddH₂O and surfactant, sonicating for 30 min to form a fine emulsion b1. Preparing a 0.1 wt% SDS solution, adding styrene at 1: 1 v/v, and the solution is emulsified to form solution b2 after being stirred evenly. Mixing b1 and b2 at 1: 3v/v, adding ddH₂O-soluble initiator and stirring for 0.5 hour, transferring to a ddH₂O bath at 75°C and reacting for 18 hours, then "magnetic composite microspheres" can be obtained. Next, adding 0.5 wt% Tween-20 solution to the above magnetic composite microspheres, sonicating for 30 minutes, discarding the supernatant after magnetic separation. The sonication/magnetic separation is performed alternately for 3 times to obtain a stable and uniformly dispersed nano-microsphere polymer suspension pre-LibNorm of 1wt%. Under the catalysis of ammonia, adding glycerol and pre-LibNorm to ethyl orthosilicate at 1: 1: 1 v/v, and reacting at room temperature for 24 hours. Washing the product with 100% ethanol/ddH₂O alternately for three times, and vacuum-drying at 40°C to obtain the final product. A hydrophilic, monodisperse, high magnetic nano magnetic composite microsphere LibNorm ("nano-microspheres" for short) is obtained. The test result indicates that the average particle diameter of the nano-microspheres is 0.5 to 2 µm.
1.2 Due to the excellent hydrophilic properties, the LibNorm magnetic microspheres can be stably dispersed as a uniform nano-microsphere suspension in a pre-prepared diluted matrix LibNorm buffer.

### Embodiment 2 Automated Nano-microsphere Dilution Gradient Verification

Diluting the nano-microspheres in embodiment 1 with the dilution gradients of: 10x, 30x, 90x, 120x, 240x, 360x, 480x, 540x, 600x, 720x, 900x (one duplicate well per gradient). Equal proportional dilution is performed on 20µL nucleic acid sample of 20ng/ µL with a programmed iNaSP automatic instrument (adding 45µL of nano-microspheres to the nucleic acid sample, shaking and capturing for 5min at room temperature, discarding the supernatant, adding 90µL of 10 mM Tris-HCL pH8.5 elution buffer, and adding the same amount of solvent), and using Qubit3.0 to detect the concentration. The results are as follows:

**Table 1 Equal proportional dilution results of LibNorm beads nano-microspheres by iNaSP automatic operation (unit: ng /µL)**

| Dilution ratio | Parallel 1 | Parallel 2 | Ave. |
|---|---|---|---|
| 10x | 6.68 | 6.72 | 6.70 |
| 30x | 5.16 | 5.08 | 5.12 |
| 90x | 3.88 | 4.12 | 4.00 |
| 120x | 4.48 | 3.98 | 4.23 |
| 240x | 2.81 | 3.05 | 2.93 |
| 360x | 2.92 | 2.46 | 2.69 |
| 480x | 2.46 | 2.77 | 2.615 |
| 540x | 2.28 | 2.44 | 2.36 |
| 600x | 2.12 | 2.44 | 2.28 |
| 720x | 1.66 | 2.02 | 1.84 |
| 900x | 2.02 | 1.85 | 1.935 |

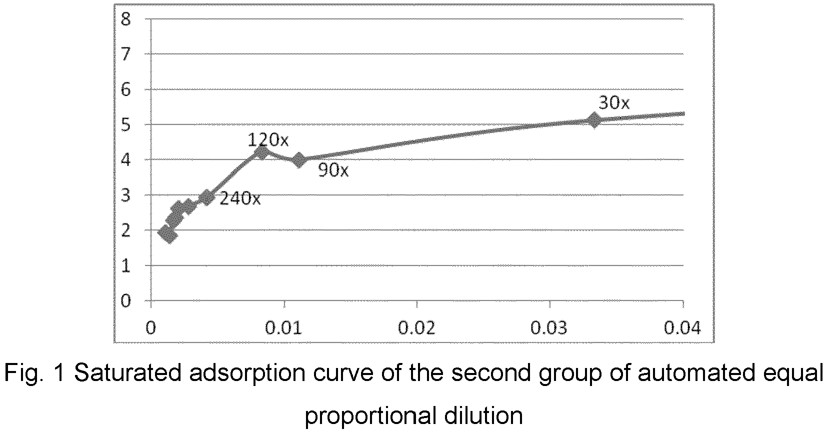

According to the results in Table 1, in the test of equal proportional diluting nucleic acid with nano-microspheres by iNaSP automatic apparatus, the saturated adsorption dilution ratio of LibNorm beads is 120x.

### Embodiment 4 Experiment of Equal Proportional Dilution of Original Samples with Different Concentrations with Nano-Microspheres Operated by iNaSP Automatic Instrument.

Using 120x-diluted nano-microspheres to verify the equal proportional dilution of 8 nucleic acids with different concentrations (Ct values ranging from small to large). Adding 45µL of nano-microspheres to each nucleic acid sample, after shaking and capturing at room temperature for 5min, discarding the supernatant, adding 90µL of 10mM Tris-HCL pH8.5 elution buffer, and then adding the same amount of solvent). Each sample employs three duplicate wells. Qubit3.0 test results are as follows:

**Table 2 Results of automated equal proportional dilution of cervical secretion nucleic acid (unit: ng / µL)**

| | iNaSP automatic operation | | | Manual operation | |
|---|---|---|---|---|---|
| Initial Ct value | Parallel 1 | Parallel 2 | Parallel 3 | Parallel 1 | Parallel 2 |
| 19.0 | 1.45 | 1.80 | 1.69 | 1.31 | 1.36 |
| 21.4 | 1.52 | 1.59 | 1.43 | 1.38 | 1.46 |
| 24.9 | 1.18 | 1.39 | 1.67 | 1.12 | 1.37 |
| 28.2 | 1.12 | 1.44 | 1.50 | 1.44 | 1.28 |
| 28.5 | 1.22 | 1.41 | 1.28 | 1.32 | 1.29 |
| 31.4 | 1.25 | 1.52 | 1.30 | 1.28 | 1.33 |
| 31.6 | 1.21 | 1.25 | 1.43 | 1.41 | 1.28 |
| 32.6 | 1.21 | 1.26 | 1.53 | 1.30 | 1.18 |

The results in Table 2 indicate that for the starting template with different concentrations, the requirement of equal proportional dilution can be achieved after the equal proportional dilution by 120x diluted nanometer microspheres (CV<0.05). That is, the iNaSP automated operating system can successfully achieve automation of LibNorm beads nano-microspheres with good stability. At the same time, the results also show that the iNaSP automated equal proportional dilution of nucleic acid (automation group, the same below) can be equivalent to manual operation.

### Embodiment 5 Equal Proportional Dilution of Nucleic Acid of Mouse Small Intestine Tissue by Libnorm Beads

1) Weighing 8 part of small intestine tissue of mice, each part has a weight of 20mg, and extracting total nucleic acid with NucleoMag® 96 Tissue;
2) Adding 90µL of distilled ddH₂O to 10µL of the nucleic acid extracted in the step 1), completing a 10-fold dilution, and obtaining a nucleic acid sample of 200ng / µL Taking 20µL of the 10-fold diluted nucleic acid, and adding 45µL of 120-fold diluted LibNorm beads nano-microspheres. After shaking and capturing for 5min at room temperature, discarding the supernatant, adding 90µL of 10 mM Tris-HCL solution with pH8.5 to elute, and adding solvent of the same amount. Finally, detecting the nucleic acid concentration after being diluted in equal proportion by using Qubit3.0;
3) non-nano-microsphere group and nano-microsphere automation group are used as control groups.

The quantitative results of Qubit3.0 are shown in Table 3.

**Table 3 Concentration detection results of nucleic acid of mouse small intestine tissue after equal proportional dilution (unit: ng / µL)**

| Serial number | Non-nano-microsphere group | Nano-microsphere group (manual operation) | Nano-microsphere automation group |
|---|---|---|---|
| 1 | 1.39 | 1.46 | 1.58 |
| 2 | 1.40 | 1.48 | 1.55 |
| 3 | 1.45 | 1.45 | 1.54 |
| 4 | 1.43 | 1.52 | 1.57 |
| 5 | 1.39 | 1.49 | 1.55 |
| 6 | 1.50 | 1.51 | 1.57 |
| 7 | 1.47 | 1.47 | 1.54 |
| 8 | 1.46 | 1.45 | 1.56 |

The results in Table 3 show that the nano-microsphere groups (manual operation groups and automation groups) can achieve the effect of diluting nucleic acids in equal proportions as the non-nano-microsphere group. The nano-microsphere group (manual operation) and nano-microsphere automation group are basically equivalent, and the latter has smaller differences in concentration between different equal proportional dilution treatments, that is, it is more stable.

### Embodiment 6 Equal Proportional Dilution Results of Human Saliva Sample Microbial 16S rDNA Community Microecological High-throughput Sequencing Library

1) Taking 200µL of freshly collected saliva and using QIAamp DNA Mini Kit to extract total nucleic acid;
2) 16S rDNA PCR: Preparing a PCR system-10 * buffer 5 µL, Mg²⁺ (25 mM) 4 µL, dNTP (10 mM) 1 µL, Taq enzyme 0.5 µL, H₂O 12.5 µL, Amplicon PCR Forward / Reverse Primer (1µM) 0.5 µL each, DNA template 1 µL (all for each person); PCR conditions are--95°C for 3 minutes; 95°C for 30 seconds, 55°C for 30 seconds, 72°C for 30 seconds, 25 cycles; another 72°C for 5 minutes.
3) The PCR products are purified by AMPure XP beads and then linked with lllumina index linkers. The linker PCR system is 10 * buffer 5 µL, Mg²⁺ (25 mM) 4 µL, dNTP (10 mM) 1 µL, Taq enzyme 0.5 µL, H₂O 24.5 µL, 5 µL each for Index 1 and Index 2, 5 µL for DNA template. The PCR condition is 95°Cfor 3 minutes; 95°C for 30 seconds, 55°Cfor 30 seconds, 72°Cfor 30 seconds, 8 cycles; another 72°C for 5 minutes;
4) Taking 20µL of sequencing library with different concentrations, respectively, and adding 45µL of 120-fold diluted LibNorm beads nano-microspheres. After shaking and capturing for 5min at room temperature, discarding the supernatant, adding 90µL of 10 mM Tris-HCL solution with pH8.5 to elute, and adding solvent of the same amount of into each sample, finally, detecting the concentration for each sequencing library that has been diluted in equal proportion by using Qubit3.0;
5) non-nano-microsphere group and nano-microsphere automation group are used as control groups.

The quantitative results of Qubit3.0 are shown in Table 4:

**Table 4 Concentration detection results of high-throughput sequencing library of saliva samples (unit: ng / µL)**

| Serial number | Non-nanospheres | Nano microsphere group (manual operation) | Nano-microsphere automation group |
|---|---|---|---|
| 1 | 1.48 | 1.60 | 1.61 |
| 2 | 1.50 | 1.59 | 1.58 |
| 3 | 1.51 | 1.55 | 1.57 |
| 4 | 1.33 | 1.54 | 1.54 |
| 5 | 1.29 | 1.44 | 1.58 |
| 6 | 1.47 | 1.52 | 1.60 |
| 7 | 1.51 | 1.55 | 1.55 |
| 8 | 1.38 | 1.46 | 1.59 |

The results in Table 4 show that the nano-microsphere groups (manual operation groups and automation groups) can achieve the effect of diluting the high throughput sequencing libraries in equal proportions as the non-nano-microsphere group. The nano-microsphere group (manual operation) and nano-microsphere automation group are basically equivalent, and the latter has smaller differences in concentration between different equal proportional dilution treatments, that is, it is more stable.

### Embodiment 7 Equal proportional dilution of nucleic acid of mouse small intestine tissue by glass beads

1) Taking 8 parts of mouse small intestine total nucleic acid with different concentrations;
2) adding 90µL of distilled ddH₂O to 10µL of the nucleic acid extracted in the step 1), completing a 10-fold dilution. Taking 20µL of 10-fold diluted nucleic acid, and adding 45µL of 150-fold diluted glass beads (diameter of 0.5 to 2µm), such that the nucleic acid samples absorbed by glass beads in each sample are saturated. After shaking and capturing for 5min at room temperature, discarding the supernatant after centrifugation at 12000rpm for 5min, adding 90µL of 10 mM Tris-HCL pH8.5 solution to elute, finally, detecting the concentration for each nucleic acid that has been diluted in equal proportion by using Qubit3.0;
3) non-glass beads group and glass beads automation group are used as control groups.

Qubit3.0 quantitative results are shown in Table 5:

**Table 5 Concentration detection results of nucleic acid of mouse small intestine tissue by equal proportional dilution by glass beads (unit: ng / µL)**

| Initial Ct value | Non-glass beads group | Glass beads group (manual operation) | Glass bead automation group |
|---|---|---|---|
| 18.0 | 1.43 | 1.47 | 1.54 |
| 20.2 | 1.42 | 1.51 | 1.57 |
| 22.5 | 1.46 | 1.49 | 1.56 |
| 26.2 | 1.44 | 1.51 | 1.54 |
| 28.1 | 1.52 | 1.54 | 1.58 |
| 30.4 | 1.49 | 1.53 | 1.56 |
| 31.8 | 1.44 | 1.49 | 1.55 |
| 33.2 | 1.46 | 1.55 | 1.54 |

The results in Table 5 show that the glass beads groups (manual operation and automation groups) can achieve the effect of diluting nucleic acids in equal proportions as the non-glass beads group. The glass beads group (manual operation) and the glass beads automation group are basically equivalent, and the latter has smaller differences in concentration between different equal proportional dilution treatments, that is, it is more stable.

The above embodiments are intended to illustrate the disclosed embodiments of the disclosure and are not understood as restrictions on the disclosure. In addition, various modifications of the present disclosure, as well as variations of the methods and compositions of the disclosure, will be apparent to those skilled in the art without departing from the scope of the disclosure. While the disclosure has been described in detail in connection with various specific preferred embodiments thereof, however, it should be understood that the present disclosure should not be limited to these specific embodiments. In fact, various modifications to the disclosure as apparent to those skilled in the art are intended to be included within the scope of the disclosure.

## Claims

1. A nucleic acid homogenization method, comprising at least the following steps:
(1) respectively adding nucleic acid adsorption materials having a same nucleic acid saturation adsorption amount into a plurality of nucleic acid solutions, wherein the nucleic acid adsorption materials added into each nucleic acid solution achieves a nucleic acid adsorption saturation state;
(2) separating the nucleic acid adsorption materials of the saturation absorbed nucleic acids;
(3) eluting the nucleic acids from the separated nucleic acid adsorption materials.

2. The nucleic acid homogenization method according to claim 1, wherein the nucleic acid adsorption materials are coated with any one or more of a carboxyl group, an amino group, a hydroxyl group, and a silicon group.

3. The nucleic acid homogenization method according to claim 1, wherein the nucleic acid adsorption materials added into each of the nucleic acid solutions is the same and has the same amount.

4. The nucleic acid homogenization method according to claim 3, wherein the nucleic acid adsorption materials are nano-microspheres or glass particles.

5. The nucleic acid homogenization method according to claim 4, wherein the nucleic acid adsorption materials are monodisperse nano-microspheres or monodisperse glass particles.

6. The nucleic acid homogenization method according to claim 3, wherein the nano-microspheres are capable of being magnetically adsorbed.

7. The nucleic acid homogenization method according to claim 3, wherein the nano-microspheres are formed by coating Fe₃O₄ with oleic acid.

8. The nucleic acid homogenization method according to claim 3, wherein an average particle diameter of the nano-microspheres is 0.5 to 2 µm.

9. The nucleic acid homogenization method according to claim 1, further comprising step (4): adding a solvent to the nucleic acid.

10. A kit for nucleic acid homogenization, wherein the kit comprises a nucleic acid adsorption material, and the nucleic acid adsorption material is coated with any one or more of a carboxyl group, an amino group, a hydroxyl group, and a silicon group.

11. The kit according to claim 10, wherein the nucleic acid adsorption material is nano-microspheres or glass particles.

12. The kit according to claim 10, wherein the kit further comprises at least one of ethanol with a volume fraction of 70-85%, ddH₂O or Tris-HCL buffer.

13. Use of the kit according to claims 10 to 12 for nucleic acid homogenization.
